(19) Europäisches Patentamt
European Patent Office
Office européen des brevets

(11) Publication number: **0 009 898**
**B1**

(12) **EUROPEAN PATENT SPECIFICATION**

(45) Date of publication of patent specification: **18.05.83**

(21) Application number: **79301854.0**

(22) Date of filing: **10.09.79**

(51) Int. Cl.³: **C 07 C 103/52,**
C 07 C 153/07,
A 61 K 37/64

(54) **Novel anti-hypertensive mercaptoacylamino acid derivatives, their preparation and use.**

(30) Priority: **11.09.78 US 941289**
**06.11.78 US 958180**
**14.08.79 US 64897**
**14.08.79 US 64898**
**14.08.79 US 64899**
**14.08.79 US 64900**
**14.08.79 US 64901**
**14.08.79 US 64902**
**14.08.79 US 64903**

(43) Date of publication of application:
**16.04.80 Bulletin 80/8**

(45) Publication of the grant of the patent:
**18.05.83 Bulletin 83/20**

(84) Designated Contracting States:
**BE CH DE FR GB LU NL SE**

(56) References cited:
**DD - A - 130 477**
**DE - A - 2 703 828**
**DE - A - 2 752 719**
**DE - A - 2 752 720**

(73) Proprietor: **UNIVERSITY OF MIAMI**
**Coral Gables Florida 33124 (US)**

(72) Inventor: **Ryan, James Walter**
**3420 Poinciana Avenue**
**Miami Florida 33133 (US)**
Inventor: **Chung, Alfred**
**8781 Southwest 87th. Street**
**Miami Florida 33183 (US)**

(74) Representative: **De Minvielle-Devaux, Ian Benedict Peter et al,**
**CARPMAELS & RANSFORD 43, Bloomsbury Square**
**London WC1A 2RA (GB)**

(56) References cited:
**DE - A - 2 753 824**
**FR - A - 2 377 374**
**US - A - 4 108 886**

**E. G. ERDÖS, Conversion of angiotensin I to angiotensin II—Am. J. Medicine 60, 749 (1976).**

**EP 0 009 898 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

Novel anti-hypertensive mercaptoacylamino acid derivatives, their preparation and use

Angiotensin converting enzyme (peptidyldipeptide hydrolase, hereinafter referred to as ACE) occupies a central role in the physiology of hypertension. The enzyme is capable of converting the decapeptide angiotensin I, having the sequence

AspArgValTyrIleHisProPheHisLeu

to an octapeptide, angiotensin II by removal of the carboxyterminal HisLeu. The symbols for various chemical entities are explained in the following table:

Ala=L-alanine
Arg=L-arginine
Asp=L-aspartic acid
<Glu=pyro-L-glutamic acid
Gly=glycine
Hip=Hippuric acid (Benzoyl-glycine)
His—L-histidine
Ile=L-isoleucine
Leu=L-leucine
Phe=L-phenylalanine
Pro=L-proline
△Pro=L-3,4-dehydroproline
Ser=L-serine
Trp=L-tryptophan
Try=L-tyrosine
Val=L-valine

ACE=Angiotensin converting enzyme
Hepes=N-2-hydroxyethylpiperazine-N'-2-ethanesulfonic acid

Angiotensin I is formed by the action of the enzyme renin, an endopeptidase found in kidney, other tissues and plasma, acting on a serum $\alpha$-2 globulin.

Blood pressure is affected by certain peptides found in the blood. One of these, angiotensin II, is a powerful pressor (blood pressure elevating) agent. Another, bradykinin, a nonapeptide with the sequence ArgProProGlyPheSerProPheArg is a powerful depressor (blood pressure lowering) agent. In addition to a direct pressor effect, angiotensin II stimulates release of aldosterone which tends to elevate blood pressure by causing retention of extracellular salt and fluids. Angiotensin II is found in measurable amount in the blood of normal humans. However, it is found at elevated concentrations in the blood of patients with renal hypertension.

The level of ACE activity is ordinarily in excess, in both normal and hypertensive humans, of the amount needed to maintain observed levels of antiogensin II. However, it has been found that significant blood pressure lowering is achieved in hypertensive patients by treatment with ACE inhibitors. [Gavras, I., et al., New Engl. J. Med. 291. 817. (1974)].

An article by Erdos. E. G., in Am. J. Medicine *60* 749 (1976) states that substrates of ACE can be represented by the general formula $R_1—R_2—R_3—OH$ and further states that $R_3$ in this formula should be an amino acid with a free carboxyl terminal, but not glutamic acid.

It is known that certain compounds having the general structure

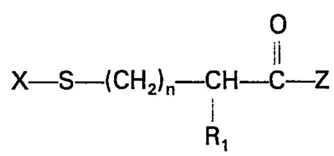

(wherein n is 0 or 1 and $R_1$ is hydrogen or methyl) are inhibitors of ACE. For example, German OLS 2752719 discloses compounds of the above formula wherein, Z may be, *inter alia,* thiazolidine carboxlic acid, and German OLS 2703828 discloses compounds wherein Z may be, *inter alia,* hydrogen, lower alkanoyl or benzoyl.

Detailed description of the invention

The present invention relates to novel inhibitors of ACE which have the general formula

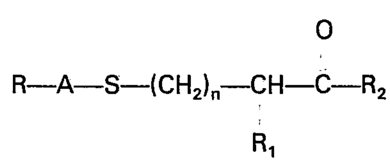

wherein R is hydrogen, formyl, acetyl, propanoyl, butanoyl, phenylacetyl, phenylpropanoyl, benzoyl, cyclopentylcarbonyl, tert-butyloxycarbonyl, cyclopentylcarbonyl-L-lysyl, pyro-L-glutamyl-L-lysyl, L-lysyl, L-arginyl or pyro-L-glutamyl;

A is L-phenylalanyl, glycyl, L-alanyl, L-tryptophyl, L-tyrosyl, L-isoleucyl, L-leucyl, L-histidyl or L-valyl, the $\alpha$-amino group thereof being in amide linkage with R;

$R_1$ is hydrogen or methyl;

$R_2$ is L-proline, L-3,4-dehydroproline, D,L-3,4-dehydroproline, L-3-hydroxyproline, L-4-hydroxyproline or L-thiazolidine carboxylic acid, the imino group thereof being in imide linkage with the adjacent

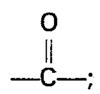

and,

n is 0 or 1, such that when n=0, $R_1$ is methyl.

All amino acids discussed herein are in the L-configuration unless otherwise noted. The disclosed compounds are inhibitors of ACE and are useful as orally effective antihypertensive agents.

The discovery of ACE inhibitor potency in the compounds of the present invention provides a unique approach to the design of inhibitory compounds. Although many prior art inhibitors are proline derivatives, substitution of other amino acids for proline has also yielded potent inhibitors. Arginine, phenyl-alanine and alanine are all effective substitutes for proline, so that a trend is not discernible.

The substitution of L-3,4-dehydroproline for proline has been studied in several systems. Substitution of L-3,4-△Pro in the 7 position of bradykinin yields a bradykinin derivative which has significantly reduced physiological activity. See Fisher, G. H. et al., *Arch. Biochem. Biophys. 189,* 81 (1978). On the other hand, substitution of L-3,4-△Pro at the 3, 5 or 9 position in ACE inhibitor $BPP_{9a}$ enhances its inhibitory activity. However, at present, no rationale can be advanced to explain the diversity of observed results following substitution of △Pro for proline. Similarly, no clear picture has emerged of the effects of other proline derivatives or analogs substituted at various loci on ACE inhibitors.

To date, the effect of the amino acid to the left of the sulfur in the above-shown formula, has not been determined. It is thought that this amino acid functions as an additional recognition site for the enzyme. If this is true, it would be expected that a compound with an amino acid here would be a better inhibitor. It was not known which, if any, amino acids would be effective in this position and which if any would enhance the inhibitory activity of a given compound. Applicants have found that various amino acids are effective and that the hydroxyprolines, proline, L-, and D,L-3,4-dehydroproline, and thiazolidine carboxylic acid derivatives are all effective anti-hypertensive agents and have high inhibitory potency for ACE.

The present invention will be further described by the following examples. In these examples, the thin-layer chromatography (TLC) was performed using silica gel plates. The numerical solvent systems for use in the TLC procedures are as follows.

(1) is methanol:chloroform, 1:1 (parts by volume). (2) is benzene:water:acetic acid, 9:1:9 (parts by volume). (3) is acetic acid:water:n-butanol 26:24:150 (parts by volume). (4) is n-butanol:pyridine:acetic acid:water, 15:10:3:12 (parts by volume). (5) is chloroform:methanol:ammonium hydroxide, 60:45:20 (parts by volume). The buffers for paper electrophoresis were pH 1.9—formic acid:acetic acid:water, 3:2:25 (parts by volume); pH 5.0—diethylene glycol:acetic acid:pyridine:water,100:6:8.5:885 (parts by volume). The tert-butyloxycarbonyl, benzoyl, acetyl, formyl, propanoyl, butanoyl, phenylacetyl and phenylpropanoyl derivatives of the amino acids are commercially available. Sephadex LH-20, Sephadex G-10 and Sephadex G-25 are trademarks of Pharmacia, Inc., Uppsala, Sweden.

Example 1

ACE activity assay

For most experiments described herein, the enzyme was assayed in 0.05 M Hepes buffer, pH 8.0 containing 0.1 M NaC1 and 0.75 M $Na_2SO_4$. The substrate employed was Benzoyl-GlyHisLeu at a final concentration of $1\times10^{-4}$M, ($K_m\approx2\times10^{-4}$M), together with about 130,000 cpm of [$^3$H]BenzoylGlyHisLeu (25 Ci/mmole). Enzyme was diluted in the above buffer such that 40 $\mu$l buffered enzyme was capable of hydrolysing 13% of substrate in a 15-minute incubation at 37°C. To initiate the assay, 40 $\mu$l of enzyme and 10 $\mu$l of water or inhibitor dissolved in water were preincubated for five minutes at 37°C. Substrate, 50 $\mu$l, was then added to initiate reaction and the solution was incubated for 15 minutes at 37°C. To terminate the reaction, 1 ml of 0.1 M HC1 was added, following which 1 ml of ethyl acetate was added. The mixture was agitated on a rotary mixer and centrifuged briefly to separate the phases.

An aliquot, 500 $\mu$l, of the ethyl acetate layer was transferred to a liquid scintillation vial containing 10 ml of Riafluor, trademark New England Nuclear Corporation, Boston, Massachusetts. For

determination of $I_{50}$ values, enzyme activity in the presence of inhibitor at a series of different concentrations was compared to activity in the absence of inhibitor. A plot of inhibitor concentration versus percent inhibition yielded the $I_{50}$ value.

Example 2

Synthesis of 3-acetylthiopropanoyl-L-proline-t-butylester

3-acetylthiopropanoic acid, 0.865 g, was dissolved in 2 ml redistilled tetrahydrofuran (THF) and cooled to 0°C. A cooled solution of dicyclohexylcarbodiimide, 1.2031 g in 2 ml of THF was added, following which a cooled solution of L-proline-t-butyl ester, 1 g, was added. The reaction mixture was stirred at 0°C for one hour, then at 4°C overnight. The reaction mixture was then filtered and the precipitate was washed with ethyl acetate. Solvents of the filtrates were removed under reduced pressure in a rotary evaporator. The residue was dissolved in ethyl acetate which was then washed three times with cold 1 N citric acid, twice with saturated NaC1, twice with cold 1 N $NaHCO_3$ and three times with saturated NaC1. The solution was dried over anhydrous $MgSO_4$ and filtered. The solvent was removed under reduced pressure in a rotary evaporator at 30°C yielding a clear colorless oily product in approximately 87% yield. The product migrated as a single spot in thin layer chromatography in five solvent systems.

Example 3

Synthesis of 3-mercaptopropanoyl-L-proline

The product from Example 2,3-acetylthiopropanoyl-L-proline-t-butyl ester, 0.5 g, was mixed with 4.5 ml of 5.5 N methanolic ammonia at room temperature under nitrogen for one hour to remove the acetyl group. The solvent was then removed at 25°C with a rotary evaporator. After the product was taken up in methanol and reevaporated twice more in the rotary evaporator, the clear oily residue was dissolved in ethyl ether, washed twice with 5% potassium bisulphate and once with saturated NaC1, dried over $MgSO_4$ and filtered. Residual solvent was removed *in vacuo* to yield a clear oily product, migrating as a single spot on thin layer chromatography in three separate solvent systems. The t-butyl ester protecting group was removed by reaction with trifluoroacetic acid in anisole.

Examples 4—6

By substituting 2-acetylthiopropanoic acid, 3-acetyl-thio-2-D-methylpropanoic acid, or 3-acetylthio-2-D,L-methylpropanoic acid for the 3-acetylthiopropanoic acid in Example 2 and substantially following the procedures of Examples 2 and 3, the following compounds are obtained. By removing the t-butyl ester protecting group with trifluoroacetic acid in anisole as a first step, the dicyclohexylamine salt can be formed to assist in the resolution of isomers. The acetyl protecting group can be removed in a second step using methanolic ammonia, as described in Example 3.

| Example | Compound |
|---|---|
| 4 | 2-mercaptopropanoyl-L-proline |
| 5 | 3-mercapto-2-D-methylpropanoyl-L-proline |
| 6 | 3-mercapto-2-D,L-methylpropanoyl-L-proline |

Example 7

Synthesis of 3-mercapto-2-methyl-propanoyl-L-3,4-dehydroproline

L-3,4-dehydroproline ($\Delta^3$Pro), 1 mmole, is dissolved in DMF and the solution is cooled to −15°C. The solution is neutralized by adding 1 equivalent of N-ethyl morpholine. In a separate reaction vessel at −10°C, one equivalent of 3-acetylthio-2-methyl-propanoic acid in an equal volume of DMF is mixed with 1.1 equivalent of 1,1′-carbonyldiimidazole, and the solution is stirred for one hour. The first solution containing $\Delta^3$Pro is mixed with the second, containing 3-acetylthio-2-methyl propanoic acid while maintaining the temperature at −10°C. The combined solution is stirred for 1 hour at −10°C. The solution is then allowed to warm slowly to room temperature. The solvent is removed on a rotary evaporator under reduced pressure at 40°C. Ethyl acetate (25 ml) is added and the solution is cooled to 0°C. Two ml of 1 N citric acid is added, the two phases are mixed and then allowed to separate. The phases are separated with a separating funnel, and the organic phase is washed twice more with 2 ml 1 N citric acid, two times with saturated NaC1 and finally dried over anhydrous $MgSO_4$. The $MgSO_4$ is removed by filtration, and the solvent is removed with a rotary evaporator. The residue is dissolved and recrystallized from a non-polar solvent such as benzene to yield 3-acetylthio-2-D,L-methylpropanoyl-L-3,4-dehydroproline. When the 2-D-methyl isomer is desired, the residue is dissolved in acetonitrile (approximately 3 ml) and the solution is warmed to 40°C. One equivalent of dicyclohexylamine is added, and the solution is allowed to stand at room temperature overnight. The crystals are collected by filtration and are washed three times with acetonitrile. When further purification is required, the material can be recrystallized from isopropanol. The acetyl protecting group can be removed as in Example 3.

Examples 8—11

By substituting D,L-3,4-dehydroproline, L-3-hydroxyproline, L-4-hydroxyproline, or L-thiazolidine carboxylic acid for the L-3,4-dehydroproline in Example 7 and substantially following the procedures of Example 7 the following compounds are obtained.

| Example | Compound |
|---|---|
| 8 | 3-mercapto-2-D-methylpropanoyl-D,L-3,4-dehydroproline |
| 9 | 3-mercapto-2-D-methylpropanoyl-L-3-hydroxyproline |
| 10 | 3-mercapto-2-D-methylpropanoyl-L-4-hydroxyproline |
| 11 | 3-mercapto-2-D-methylpropanoyl-L-thiazolidine |

Example 12

Similarly, by substituting 3-acetylthiopropanoic acid or 2-acetylthiopropanoic acid for the 3-acetylthio-2-methyl propanoic acid of Examples 7—11, the L-3,4-dehydroproline, D,L-3,4-dehydroproline, L-3-hydroxyproline, L-4-hydroxyproline and L-thiazolidine derivatives are obtained, following substantially the described procedures.

Example 13

Synthesis of $N^{\alpha}$-[3-($N^{\alpha}$-acetyl-L-phenylananylthio)-2-D-methylpropanoyl]-L-proline

A solution of 41.5 mg of $N^{\alpha}$-acetyl-L-phenylalanine in 0.5 ml redistilled dimethylformamide (DMF) was cooled in an ice-dry ice-acetone bath at −20°C. To this solution was added a cold solution of 35 mg of 1,1′-carbonyldiimidazole in 1.0 ml of DMF. The solution was stirred at −10°C for two hours and then was added to a cold solution of 48 mg of 3-mercapto-2-D-methylpropanoyl-L-proline in 1 ml of DMF which was neutralized with N-ethyl morpholine. The reaction mixture was stirred at −10°C for an additional hour and then slowly warmed to room temperature. The solvent was removed under reduced pressure at 40°C and ethyl acetate was added to the residue. The mixture was cooled in an ice bath and washed with 0.1 N HC1 and then three times with saturated NaC1 solution. The organic solvent was removed with a rotary evaporator after drying over anhydrous $MgSO_4$. The product was purified by Sephadex LH-20[tm] column chromatography using a 1.2 cm by 95 cm column and eluted with isopropanol. The peak fractions were pooled and the solvent was removed under reduced pressure yielding 35.5 mg of the named product. This product was found to be homogeneous using TLC with solvent systems 1, 2, 3 and 5.

Examples 14—21

By substituting $N^{\alpha}$-acetyl-glycine, $N^{\alpha}$-acetyl-alanine, $N^{\alpha}$-acetyl-tryptophan, $N^{\alpha}$-acetyl-tyrosine, $N^{\alpha}$-acetyl-isoleucine, $N^{\alpha}$-acetyl-leucine, $N^{\alpha}$-acetylhistidine, or $N^{\alpha}$-acetyl-valine for the $N^{\alpha}$-acetyl-phenylalanine in Example 13 and substantially following the procedure of Example 13, the following compounds are obtained.

| Example | Compound |
|---|---|
| 14 | $N^{\alpha}$-[3-($N^{\alpha}$-acetylglycylthio)-2-D-methylpropanoyl]-L-proline |
| 15 | $N^{\alpha}$-[3-($N^{\alpha}$-acetyl-L-tryptophylthio)-2-D-methyl-propanoyl]-L-proline |
| 16 | $N^{\alpha}$-[3-($N^{\alpha}$-acetyl-L-tyrosylthio)-2-D-methyl-propanoyl]-L-proline |
| 17 | $N^{\alpha}$-[3-($N^{\alpha}$-acetyl-L-isoleucylthio)-2-D-methyl-propanoyl]-L-proline |
| 18 | $N^{\alpha}$-[3-($N^{\alpha}$-acetyl-L-leucylthio)-2-D-methylpropanoyl]-L-proline |
| 19 | $N^{\alpha}$-[3-($N^{\alpha}$-acetyl-L-histidylthio)-2-D-methylpropanoyl]-L-proline |
| 20 | $N^{\alpha}$-[3-($N^{\alpha}$-acetyl-L-valylthio)-2-D-methyl-propanoyl]-L-proline |
| 21 | $N^{\alpha}$-[3-($N^{\alpha}$-acetyl-L-alanylthio)-2-D-methyl-propanoyl]-L-proline. |

Example 22

Similarly, the L-3,4-dehydroproline, D,L-3,4-dehydroproline, L-3-hydroxyproline, L-4-hydroxyproline and L-thiazolidine carboxylic acid derivatives are obtained by substituting the products of Examples 3—12 for the 3-mercapto-2-D-methylpropanoyl-L-proline in Examples 13—21 and substantially following the procedure of Example 13.

Example 23

By substituting the $N^{\alpha}$-formyl, $N^{\alpha}$-propanoyl, $N^{\alpha}$-butanoyl, $N^{\alpha}$-phenylacetyl or $N^{\alpha}$-phenylpropanoyl derivatives of L-Phe, Gly, L-Ala, L-Trp, L-Tyr, L-Ile, L-Leu, L-His and L-Val for the $N^{\alpha}$-acetyl derivatives, in Examples 13—22 and substantially following the procedure of Example 13, the formyl, propanoyl, butanoyl, phenylacetyl, and phenylpropanoyl derivatives are obtained.

Example 24

Synthesis of $N^{\alpha}$-[3-($N^{\alpha}$-tert-butyloxycarbonyl-L-phenylalanylthio)-2-D-methylpropanoyl]-L-proline

A solution of 133 mg of $N^{\alpha}$-tert-butyloxycarbonyl-L-phenylalanine ($N^{\alpha}$-Boc-L-Phe) in 0.5 ml redistilled DMF was cooled in an ice-dry ice-acetone bath at −20°C. To this solution was added a cold

solution of 87 mg of 1,1′-carbonyldiimidazole in 1.0 ml of DMF. The solution was stirred at −10°C for two hours and then was added to a cold solution of 119.5 mg of 3-mercapto-2-D-methylpropanoyl-L-proline in 1 ml of DMF which was neutralized with N-ethyl morpholine. The reaction mixture was neutralized with N-ethyl morpholine. The reaction mixture was stirred at −10°C for an additional hour and then slowly warmed to room temperature. The solvent was removed under reduced pressure at 40°C and ethyl acetate was added to the residue. The mixture was cooled in an ice bath and washed with 0.1 N HC1 and then three times with saturated NaC1 solution. The solvent was removed with a rotary evaporator after drying over anhydrous $MgSO_4$. The product was purified by liquid chromatography on Sephadex G-10[tm] using a 1.2 cm by 95 cm column and eluted with THF:isopropanol, 3:7 (parts by volume). The peak fractions were pooled and the solvent removed under reduced pressure yielding 165 mg of the named product. This product was found to be homogeneous using paper electrophoresis at pH 5.0 and using TLC with solvent systems 1, 2 and 3.

Examples 25—32

By substituting $N^\alpha$-Boc-glucine, $N^\alpha$-Boc-alanine, $N^\alpha$-Boc-tryptophan, $N^\alpha$-Boc-tyrosine, $N^\alpha$-Boc-isoleucine, $N^\alpha$-Boc-leucine, $N^\alpha$-Boc-histidine or $N^\alpha$-Boc-valine for the $N^\alpha$-Boc-Phe in Example 24 and substantially following the procedures of Example 24, the following compounds are obtained.

| Example | Compound |
|---|---|
| 25 | $N^\alpha$-[3-($N^\alpha$-tert-butyloxycarbonylglycylthio)-2-D-methylpropanoyl]-L-proline |
| 26 | $N^\alpha$-[3-($N^\alpha$-tert-butyloxy-carbonyl-L-tryptophyl-thio)-2-D-methyl-propanoyl]-L-proline |
| 27 | $N^\alpha$-[3-($N^\alpha$-tert-butyloxycarbonyl-L-tyrosylthio)-2-D-methyl-propanoyl]-L-proline |
| 28 | $N^\alpha$-[3-($N^\alpha$-tert-butyloxycarbonyl-L-isoleucylthio)-2-D-methyl-propanoyl]-L-proline |
| 29 | $N^\alpha$-[3-($N^\alpha$-tert-butyloxy-carbonyl-L-leucyl-thio)-2-D-methyl-propanoyl]-L-proline |
| 30 | $N^\alpha$-[3-($N^\alpha$-tert-butyloxy-carbonyl-L-histidyl-thio)-2-D-methylpropanoyl]-L-proline |
| 31 | $N^\alpha$-[3-($N^\alpha$-tert-butyloxycarbonyl-L-valylthio)-2-D-methyl-propanoyl]-L-proline |
| 32 | $N^\alpha$-[3-($N^\alpha$-tert-butyloxy-carbonyl-L-alanylthio)-2-D-methyl-propanoyl]-L-proline. |

Example 33

Similarly, the L-3,4-dehydroproline, D,L-3,4-dehydroproline, L-3-hydroxyproline, L-4-hydroxyproline and L-thiazolidine carboxylic acid derivatives are obtained by substituting the products of Examples 3—12 for the 3-mercapto-2-D-methylpropanoyl-L-proline in Examples 24—32 and substantially following the procedure in Example 24.

Example 34

Synthesis of $N^\alpha$-cyclopentylcarbonyl-L-phenylalanine

A cool solution of 2.06 gm of dicyclohexylcarbodiimide in 10 ml of dichloromethane was added to a solution of 1.4114 gm of cyclopentanecarboxylic acid in 5 ml of dichloromethane at −5°C. It was followed by the addition of 4.28 gm of L-phenylalanin benzoyl ester toluenesulfonate salt in 10 ml of DMF which was neutralized with 1.36 ml of N-ethyl morpholine. The reaction mixture was stirred at 0°C for one hour and then at room temperature for three hours. Dicyclohexylurea was removed by filtration and 50 ml of ethyl acetate was added to the filtrate. The organic phase was washed until neutral, dried over anhydrous $MgSO_4$ and filtered. The solvent was removed with a rotary evaporator. The residue was crystallized from isopropanol and hexane yielding 2.35 gm of white crystals having a melting point of 88—89°C. Elemental analysis of these crystals yielded the following.

Calculated: C=75.19; H=7.17; N=3.9855
Found: C=74.96; H=7.17; N=4.09.

The benzyl ester was removed by hydrogenolysis with 2 gm of 10% palladium on carbon in absolute alcohol. The catalyst was removed by filtration and the ethanol was removed by a rotary evaporator. The residue was crystallized from ether and hexane yielding 1.15 gm white crystals of the named product having a melting point of 107—108°C. Elemental analysis of these crystals yielded the following.

Calculated: C=68.94; H=7.33; N=5.36
Found: C=68.90; H=7.32; N=5.34.

The product was found to be homogeneous using paper electrophoresis at pH 1.9 and at pH 5.0 and using TLC with solvent systems, 1, 2 and 3. The named product may be abbreviated as $N^\alpha$-cpc-L-Phe.

Example 35

Synthesis of $N^\alpha$-[3-($N^\alpha$-cyclopentylcarbonyl-L-phenyl-alanylthio)-2-D-methylpropanoyl]-L-proline

A solution of 52.5 mg of the compound from Example 34 in 0.5 ml redistilled DMF was cooled in an ice-dry ice-acetone bath at −20°C. To this solution was added a cold solution of 34 mg of 1,1′-carbonyldiimidazole in 1.0 ml of DMF. The solution was stirred at −10°C for two hours and then mixed

with a cold solution of 45.6 mg of 3-mercapto-2-D-methylpropanoyl-L-proline in 1 ml of DMF which was neutralized with N-ethyl morpholine. The reaction mixture was stirred at −10°C for an additional hour and then slowly warmed to room temperature. The solvent was removed under reduced pressure at 40°C and ethyl acetate was added to the residue. The mixture was cooled and washed with 0.1 N HC1 and then three times with saturated NaC1 solution. The solvent was removed with a rotary evaporator after drying over anhydrous $MgSO_4$. The product was purified by Sephadex LH-20[tm] column chromatography using a 1.2 cm by 95 cm column and eluted with isopropanol. The peak fractions were pooled and the solvent was removed under reduced pressure yielding 60.5 mg of the named product. This produce was found to be homogeneous using TLC in solvent systems 1, 2, 3 and 5.

Examples 36—43

By substituting the benzoyl ester toluenesulfonate salts of glycine, L-alanine, L-tryptophan, L-tyrosine, L-isoleucine, L-leucine, L-histidine or L-valine for the L-Phe salt in Example 34 and substantially following the procedures of Examples 34 and 35, the following compounds are obtained.

| Example | Compound |
|---|---|
| 36 | $N^\alpha$-[3-($N^\alpha$-cyclopentylcarbonyl-glycylthio)-2-D-methyl-propanoyl]-L-proline |
| 37 | $N^\alpha$-[3-($N^\alpha$-cyclopentylcarbonyl-L-tryptophylthio)-2-D-methylpropanoyl]-L-proline |
| 38 | $N^\alpha$-[3-($N^\alpha$-cyclopentylcarbonyl-L-tyrosylthio)-2-D-methyl-propanoyl]-L-proline |
| 39 | $N^\alpha$-[3-($N^\alpha$-cyclopentyl-carbonyl-L-isoleucyl-thio)-2-D-methyl-propanoyl]-L-proline |
| 40 | $N^\alpha$-[3-($N^\alpha$-cyclopentyl-carbonyl-L-leucyl-thio)-2-D-methyl-propanoyl]-L-proline |
| 41 | $N^\alpha$-[3-($N^\alpha$-cyclopentylcarbonyl-L-histidyl-thio)-2-D-methyl-propanoyl]-L-proline |
| 42 | $N^\alpha$-[3-($N^\alpha$-cyclopentylcarbonyl-L-valylthio)-2-D-methyl-propanoyl]-L-proline |
| 43 | $N^\alpha$-[3-($N^\alpha$-cyclopentylcarbonyl-L-alanylthio)-2-D-methyl-propanoyl]-L-proline. |

Example 44

Similarly, the L-3,4-dehydroproline, D,L-3,4-dehydroproline, L-3-hydroxyproline, L-4-hydroxyproline and L-thia zolidine carboxylic acid derivatives are obtained by substituting the products of Examples 3—12 for the 3-mercapto-2-D-methylpropanoyl-L-proline in Examples 35—43 and substantially following the procedure of Example 35.

Example 45

Synthesis of $N^\alpha$-(3-L-phenylalanylthio-2-D-methylpropanoyl)-L-proline

The product from Example 24 was deprotected by stirring a mixture of 30 mg of the product, 50 μl of anisole and 200 μl of anhydrous trifluoroacetic acid (TFA) at room temperature for one hour. Anisole and TFA were removed under reduced pressure at 35°C and the residue was triturated with anhydrous ether. The white residue was purified by liquid chromatography on Sephadex G-10[tm] using a 1.2 cm by 95 cm column and eluted with 5% acetic acid. The peak fractions were pooled and freeze-dried yielding 17.5 mg of the named product. This product was found to be homogeneous using paper electrophoresis at pH 1.9 and 5.0 and using TLC with solvent systems 4 and 5.

Examples 46—53

Similarly, by substantially following the procedure of Example 45, the products from Examples 25—32 are deprotected and the following compounds are obtained.

| Example | Compound |
|---|---|
| 46 | $N^\alpha$-(3-glycylthio-2-D-methylpropanoyl)-L-proline |
| 47 | $N^\alpha$-(3-L-tryptophylthio-2-D-methyl-propanoyl)-L-proline |
| 48 | $N^\alpha$-(3-L-tyrosylthio-2-D-methylpropanoyl)-L-proline |
| 49 | $N^\alpha$-(3-L-isoleucylthio-2-D-methylpropanoyl)-L-proline |
| 50 | $N^\alpha$-(3-L-leucylthio-2-D-methylpropanoyl)-L-proline |
| 51 | $N^\alpha$-(3-L-histidylthio-2-D-methylpropanoyl)-L-proline |
| 52 | $N^\alpha$-(3-L-valylthio-2-D-methylpropanoyl)-L-proline |
| 53 | $N^\alpha$-(3-L-alanylthio-2-methylpropanoyl)-L-proline |

Example 54

Similarly, the L-3,4-dehydroproline, D,L-3,4-dehydroproline, L-3-hydroxyproline, L-4-hydroxyproline, and L-thiazolidine carboxylic acid derivatives are obtained by deprotecting the compounds described in Example 33. The deprotection is done by substantially following the procedure of Example 45.

Example 55

Synthesis of the N-hydroxysuccinimide ester of cyclopentane carboxylic acid

A cool solution of 11.4 gm of dicyclohexylcarbodiimide in DMF was added drop-wise to a mixture of 5.71 gm of cyclopentanecarboxylic acid and 5.76 gm of N-hydroxysuccinimide in DMF at 0°C. The

reaction mixture was stirred at 0°C for 30 minutes and then at 4°C overnight. Crystalline dicyclohexylurea was removed by filtration and the precipitate was washed with ethyl acetate. Solvents from the combined filtrate were removed under reduced pressure and the residue was crystallized from benzene and hexane yielding 5.77 gm of white crystals having a melting point of 72.5°—73°C. The infrared absorption spectrum in chloroform gave a typical spectrum of N-hydroxysuccinimide esters. Elemental analysis of the crystals yielded the following results.

Calculated: C=56.86; H=6.20; N=6.63
Found: C=56.77; H=6.07; N=6.66.

Example 56
Synthesis of $N^{\alpha}$-cyclopentylcarbonyl-$N^{\varepsilon}$-tert-butyloxycarbonyl-L-lysine

A solution of 1.2316 gm of $N^{\varepsilon}$-tert-butyloxycarbonyl-L-lysine and 420 mg of $NaHCO_3$ in 10 ml of water and 5 ml of THF was cooled in an ice bath with stirring. To this solution was added a cold solution of 1.19 gm of the product from Example 55 in 10 ml of THF. The THF was removed with a rotary evaporator at 35°C after the reaction mixture had been stirred overnight at room temperature. About 20 ml of water was added to the reaction mixture and the pH was adjusted to 9 with solid $NaHCO_3$. The aqueous phase was extracted three times with ethyl acetate and the organic phase was discarded. The aqueous solution was cooled in an ice bath and then acidified to pH 2 with 1 N HC1 in the presence of ethyl acetate. The organic phase was washed twice with ice water and then twice with a solution of saturated NaC1. The organic solution was dried over anhydrous $MgSO_4$ and then filtered. The solvent was removed with a rotary evaporator and the residue was crystallized from ether and hexane yielding 1.135 gm of white crystals having a melting point of 104.5°—105°C. Elemental analysis of the crystals yielded the following.

Calculated: C=59.63; H=8.83; N=8.18
Found: C=59.74; H=8.85; N=8.24.

The product was shown to be homogeneous using paper electrophoresis at pH 1.9 and pH 5.0 and using TLC with solvent systems 1, 2 and 3.

Example 57
Synthesis of $N^{\alpha}$-cyclopentylcarbonyl-$N^{\varepsilon}$-tert-butyloxy-carbonyl-L-lysine-N-hydroxysuccinimide ester

A solution of 1.027 gm of the product from Example 56 and 346 mg of N-hydroxysuccinimide in 10 ml of $CH_2Cl_2$ was cooled to −5°C. To this solution was added with stirring a cold solution of 680 mg of dicyclohexylcarbodiimide in 5 ml of $CH_2Cl_2$. The reaction mixture was stirred at 0°C for 30 minutes and then at 4°C overnight. Crystalline dicyclohexylurea was removed by filtration and was washed with ethyl acetate. The combined filtrate was washed twice with 1 N $NaHCO_3$, twice with water and finally with a solution of saturated NaC1. The organic phase was dried over anhydrous $MgSO_4$, filtered, and the solvent was removed with a rotary evaporator. The residue was crystallized from isopropanol yielding 0.844 gm of white crystals having a melting point of 140.5°C. The infrared adsorption spectrum in chloroform gave a typical spectrum of N-hydroxysuccinimide esters. Elemental analysis of the crystals yielded the following.

Calculated: C=57.39; H=7.57; N=9.56
Found: C=57.10; H=7.57; N=9.61.

Example 58
Synthesis of $N^{\alpha}$-cyclopentylcarbonyl-$N^{\varepsilon}$-tert-butyloxycarbonyl-L-lysyl-L-phenylalanine

A solution of 220 mg of the product from Example 57 in 2 ml of dioxane was added drop-wise to a mixture of 99.1 mg of L-phenylalanine and 51 mg of $NaHCO_3$ in a mixture of 2 ml of water and 1 ml of DMF. The reaction mixture was stirred at room temperature overnight and dioxane was removed with a rotary evaporator at 35°C. Ethyl acetate (10 ml) was added to the reaction mixture, cooled, acidified at pH 2 with 0.1 N HC1 and the aqueous solution was discarded. The organic phase was washed with ice water, a solution of saturated NaC1, and dried over anhydrous $MgSO_4$; and the solvent was removed with a rotary evaporator. The residue was crystallized from ether and petroleum ether (b.p. 30—60°C) yielding 95 mg of white solid having a melting point of 90—92°C. The product was shown to be homogeneous using paper electrophoresis at pH 1.9 and pH 5.0 and using TLC with solvent systems 1, 2, 3 and 4. Elemental analysis yielded,

Calculated: C=63.78; H=8.03; N=8.58
Found: C=63.40; H=8.07; N=8.34.

Example 59
Synthesis of $N^{\alpha}$-[3-($N^{\alpha}$-cyclopentylcarbonyl-$N^{\varepsilon}$-tert-butyloxycarbonyl-L-lysyl-L-phenylalanylthio)-2-D-methylpropanoyl]-L-proline

A solution of 73.5 mg of the product from Example 58 in 0.5 ml redistilled DMF was cooled in an ice-dry ice-acetone bath at −20°C. To this solution was added a cold solution of 26 mg of 1,1′-carbonyldiimidazole in 1.0 ml of DMF. The solution was stirred at −10°C for two hours and then mixed

with a cold solution of 36 mg of 3-mercapto-2-D-methylpropanoyl-L-proline in 1 ml of DMF which was neutralized with N-ethyl morpholine. The reaction mixture was stirred at −10°C for an additional hour and then slowly warmed to room temperature. The solvent was removed under reduced pressure at 40°C and ethyl acetate was added to the residue. The mixture was cooled in an ice water bath and washed with 1 N citric acid and then three times with saturated NaC1 solution. The solvent was removed with a rotary evaporator after drying over anhydrous $MgSO_4$. The product was purified by Sephadex LH-20[tm] column chromatography using a 1.2 cm by 95 cm column and eluted with THF:isopropanol, 3:7 (parts by volume). The peak fractions were pooled and the solvent was removed under reduced pressure yielding the named product which may be abbreviated as $N^{\alpha}$-[3-($N^{\alpha}$-cpc-$N^{\epsilon}$-Boc-L-Lys-L-Phe-thio)-2-D-methylpropanoyl]-L-proline.

Example 60

Synthesis of $N^{\alpha}$-[3-($N^{\alpha}$-cyclopentylcarbonyl-L-lysyl-L-phenylalanylthio)-2-D-methylpropanoyl]-L-proline

The $N^{\epsilon}$-Boc group was removed from the lysine by stirring a mixture of 30 mg of the product from Example 59 with 50 μl anisole and 200 μl of anhydrous trifluoroacetic acid (TFA) at room temperature for one hour. Anisole and TFA were removed under reduced pressure at 35°C and the residue was triturated with anhydrous ether. The residue was purified by liquid chromatography on Sephadex G-10[tm] using a 1.2 cm by 95 cm column and eluted with 5% acetic acid. The peak fractions were pooled and freeze-dried yielding the named product which can be abbreviated as $N^{\alpha}$-[3-($N^{\alpha}$-cpc-L-Lys-L-Phe-thio)-2-D-methylpropanoyl]-L-proline.

Examples 61—68

By substituting glycine, L-alanine, L-tryptophan, L-tyrosine, L-isoleucine, L-leucine, L-histidine, or L-valine for the L-phenylalanine in Example 58 and substantially following the procedures of Examples 58, 59 and 60, the following compounds are obtained.

| Example | Compound |
|---|---|
| 61 | $N^{\alpha}$-[3-($N^{\alpha}$-cyclopentyl -carbonyl-L-lysyl-glycylthio)-2-D-methyl-propanoyl]-L-proline |
| 62 | $N^{\alpha}$-[3-($N^{\alpha}$-cyclopentylcarbonyl-L-lysyl-L-tryptophylthio)-2-D-methyl-propanoyl]-L-proline |
| 63 | $N^{\alpha}$-[3-($N^{\alpha}$-cyclopentyl-carbonyl-L-lysyl-L-tyrosylthio)-2-D-methyl-propanoyl]-L-proline |
| 64 | $N^{\alpha}$-[3-($N^{\alpha}$-cyclopentylcarbonyl-L-lysyl-L-iso-leucylthio)-2-D-methyl-propanoyl]-L-proline |
| 65 | $N^{\alpha}$-[3-($N^{\alpha}$-cyclopentyl-carbonyl-L-lysyl-L-leucylthio)-2-D-methyl-propanoyl]-L-proline |
| 66 | $N^{\alpha}$-[3-($N^{\alpha}$-cyclopentylcarbonyl-L-lysyl-L-histidylthio)-2-D-methyl-propanoyl]-L-proline |
| 67 | $N^{\alpha}$-[3-($N^{\alpha}$-cyclopentyl-carbonyl-L-lysyl-L-valylthio)-2-D-methyl-propanoyl]-L-proline |
| 68 | $N^{\alpha}$-[3-($N^{\alpha}$-cyclopentylcarbonyl-L-lysyl-L-alanylthio)-2-D-methyl-propanoyl]-L-proline. |

Example 69

Similarly, the L-3,4-dehydroproline, D,L-3,4-dehydroproline, L-3-hydroxyproline, L-4-hydroxyproline and L-thiazolidine carboxylic acid derivatives are obtained by substituting the products of Examples 3—12 for the 3-mercapto-2-D-methylpropanoyl-L-proline in Examples 59 and 61—68 and substantially following the procedures of Examples 59 and 60.

Example 70

By substituting pyro-L-glutamic acid for the cyclopentanecarboxylic acid in Example 55 and substantially following the procedures of Examples 55—57, $N^{\alpha}$-pyro-L-glutamyl-$N^{\epsilon}$-tert-butyloxycarbonyl-L-lysine-N-hydroxysuccinimide ester is obtained. By substituting this product for the $N^{\alpha}$-cpc-$N^{\epsilon}$-Boc-L-Lys-N-hydroxysuccinimide ester in Example 58 and substantially following the procedures of Examples 58—69, the pyro-L-glutamyl derivatives are obtained.

Example 71

Preparation of pyro-L-glutamyl-L-phenylalanine benzyl ester

A solution of 0.52 g of pyro-L-glutamic acid, 1.72 g of L-phenylalanine benzyl ester toluenesulfonic acid and 0.55 ml of N-ethylmorpholine in 5 ml of DMF and 20 ml of dichloromethane was cooled in an ice bath with stirring. A solution of 0.826 g of dicyclohexylcarbodiimide in 2 ml dichloromethane was added to the above reaction mixture. The reaction mixture was stirred in an ice water bath for 1 hour and then at room temperature overnight. Dicyclohexylurea was removed by filtration and the product was washed in ethyl acetate. Solvents of the combined filtrates were removed under reduced pressure with a rotary evaporator at 40°C. Ethyl acetate (25 ml) was added to the residue and the organic solution was washed until neutral. The organic phase was dried over anhydrous $MgSO_4$, filtered and then the solvent was removed with a rotary evaporator. The material was crystallized from isopropanol and ether, yield; 1.01 g of white needles, m.p. 103—104.5°C. The material was homogeneous on all TLC and electrophoresis systems. Elemental analysis yielded,

Calculated: C=68.84; H=6.05; N=7.64

Found: C=68.58; H=6.05; N=7.56.

Example 72

Preparation of pyro-L-glutamyl-L-phenylalanine

The benzoyl ester protecting group of the compound of Example 71 (1.0 g) was removed by catalytic hydrogenolysis with 150 mg of 10% (by weight) Pd on carbon in 0.15 ml of glacial acid and 15 ml of ethanol at 20 psi of $H_2$ at room temperature overnight. The catalyst was removed by filtration. Solvent was removed with a rotary evaporator. The material was crystallized from isopropanol and benzene, to yield a total of 402 mg of white crystals, m.p. 147—149°C. The material was homogeneous on electrophoresis at pH 1.9 and pH 5.0 and on TLC in systems 1, 2 and 3. Elemental analysis yielded,

Calculated: C=60.86; H=5.84; N=10.14

Found: C=60.37; H=5.85; N=9.98.

Example 73

Preparation of $N^{\alpha}$-[3-(Pyro-L-glutamyl-L-phenyl-alanylthio)-2-D-methylpropanoyl]-L-proline

A solution of 87 mg of 1,1′-carbonyldiimidazole in 1.0 ml DMF was added to a solution of 139 mg of the product of Example 72 in 0.5 ml DMF at −15°C. The reaction mixture was stirred at −10°C for 1 hour, and then a mixture of 119.5 mg of 3-mercapto-2-D-methylpropanoyl-L-proline and 0.072 ml of N-ethyl morpholine in 1 ml. DMF was added. The reaction mixture was stirred at −10°C for an additional hour and then was slowly warmed to room temperature. DMF was removed under reduced pressure with a rotary evaporator at 40°C and then 7 ml ethyl acetate and 2 ml 1 N citric acid were added. The organic phase was washed two times with 1 N citric acid and two times with saturated NaC1. The organic phase was dried with anhydrous $MgSO_4$ and then filtered. Solvent was removed using a rotary evaporator. The residue was purified on Sephadex G-25[tm] (1.2×99 cm) partition column chromatography with n-butanol:acetic acid: $H_2O$ (4:1:5 by volume). The product was homogeneous on TLC (systems 1, 2 and 3) and on electrophoresis at pH 5.0.

Example 74

By substituting $N^{\alpha}$,$N^{\epsilon}$-bis-t-butyloxycarbonyl-L-lysine (hereinafter bis-Boc-L-Lys) or $N^{\alpha}$,$N^{\gamma}$,$N^{\omega}$-triadamantyloxycarbonyl-L-arginine (hereinafter tri-Adoc-L-arginine) for pyro-L-glutamic acid and by following substantially the procedure of Example 71, the corresponding bis-Boc-L-Lys or tri-Adoc-L-Arg derivatives of L-Phe-benzyl ester will be synthesized. Bis-Boc-L-Lys is commercially available. Tri-Adoc-L-Arg is prepared according to Jager, G and Geiger, R., *Chem. Ber. 103,* 1727 (1970).

Example 75

By substituting the benzyl esters of Gly, Ala, Trp, Try, Ile, Leu, His or Val, in the procedures of Examples 71 and 74, the corresponding pyro-L-glutamyl, bis-Boc-L-Lys and tri-Adoc-L-Arg derivatives are obtained. The foregoing benzyl esters are commercially available. Benzyl ester protecting groups are removed essentially as described in Example 72.

Example 76

The compounds resulting from Examples 72, 74 and 75 are reacted with any of the compounds resulting from the procedures of Examples 3—12, following essentially the procedures of Example 73, to yield the corresponding bis-Boc-L-Lys and tri-Adoc-L-Arg derivatives. The bis-Boc and tri-Adoc protecting groups are removed by treatment with trifluoroacetic acid in anisole.

Following essentially the procedures and methods of Examples 2—12 and 71—76, a family of thiolester compounds is obtained which have the general formula

$$R—A—S(CH_2)_n—\underset{\displaystyle R_1}{\underset{|}{CH}}—\overset{\displaystyle O}{\overset{\|}{C}}—R_2$$

wherein,

R is L-arginyl, L-lysyl or pyro-L-glutamyl;

A is L-phenylalanyl, glycyl, L-alanyl, L-tryptophyl, L-tyrosyl, L-isoleucyl, L-leucyl, L-histidyl, or L-valyl whose $\alpha$-amino group is in amide linkage with R;

$R_1$ is hydrogen or methyl;

$R_2$ is L-proline, L-3,4-dehydroproline, D,L-3,4-dehydroproline, L-3-hydroxyproline, L-4-hydroxyproline or L-thiazolidine carboxylic acid whose imino group is in imide linkage with the

$$—\overset{\displaystyle O}{\overset{\|}{C}}—;$$

and n is 0 or 1 such as when n=0, $R_1$ is methyl.

Example 77

Synthesis of $N^{\alpha}$-[3-($N^{\alpha}$-benzoyl-L-tryptophylthio)-2-D-methylpropanoyl]-L-proline

A solution of 62 mg of $N^{\alpha}$-benzoyl-L-tryptophan in 0.5 ml redistilled dimethylformamide (DMF) was cooled in a ice-dry ice-acetone bath at −20°C. To this solution was added a cold solution of 35 mg of 1,1′-carbonyldiimidazole in 1.0 ml of DMF. The solution was stirred at −10°C for two hours and then added to a cold solution of 48 mg of 3-mercapto-2-D-methylpropanoyl-L-proline in 1 ml of DMF which was neutralized with N-ethyl morpholine. The reaction mixture was stirred at −10°C for an additional hour and then slowly warmed to room temperature. The solvent was removed under reduced pressure at 40°C and ethyl acetate was added to the residue. The mixture was cooled in an ice bath and washed with 0.1 N HC1 and then three times with saturated NaC1 solution. The solvent was removed with a rotary evaporator after drying over anhydrous $MgSO_4$. The product was purified by liquid chromatography on Sephadex LH-20[tm] using a 1.2 cm by 95 cm column and eluted with isopropanol. The peak fractions were pooled and the solvent removed under reduced pressure yielding 60.5 mg of the named product, as a foam-like material. This product was found to be homogeneous using TLC with solvent systems 1, 2, 3 and 5.

Examples 78—85

By substituting $N^{\alpha}$-benzoyl-L-tyrosine, $N^{\alpha}$-benzoyl-L-histidine, $N^{\alpha}$-benzoyl-L-phenylalanine, $N^{\alpha}$-benzoylglycine, $N^{\alpha}$-benzoyl-L-alanine, $N^{\alpha}$-benzoyl-L-isoleucine, $N^{\alpha}$-benzoyl-L-leucine or $N^{\alpha}$-benzoyl-L-valine for the $N^{\alpha}$-benzoyl-L-tryptophan in Example 77 and substantially following the procedures of Example 77, the following compounds are obtained:

| Example | Compound |
|---|---|
| 78 | $N^{\alpha}$-[3-($N^{\alpha}$-benzoyl-L-tyrosylthio)-2-D-methyl-propanoyl]-L-proline |
| 79 | $N^{\alpha}$-[3-($N^{\alpha}$-benzoyl-L-histidylthio)-2-D-methyl-propanoyl]-L-proline |
| 80 | $N^{\alpha}$-[3-($N^{\alpha}$-benzoyl-L-phenyl-alanylthio)-2-D-methyl-propanoyl]-L-proline |
| 81 | $N^{\alpha}$-[3-($N^{\alpha}$-benzoyl-glycylthio)-2-D-methyl-propanoyl]-L-proline |
| 82 | $N^{\alpha}$-[3-($N^{\alpha}$-benzoyl-L-alanylthio)-2-D-methyl-propanoyl]-L-proline |
| 83 | $N^{\alpha}$-[3-($N^{\alpha}$-benzoyl-L-isoleucylthio)-2-D-methyl-propanoyl]-L-proline |
| 84 | $N^{\alpha}$-[3-($N^{\alpha}$-benzoyl-L-leucylthio)-2-D-methyl-propanoyl]-L-proline |
| 85 | $N^{\alpha}$-[3-($N^{\alpha}$-benzoyl-L-valylthio)-2-D-methyl-propanoyl]-L-proline. |

Example 86

Similarly, the L-3,4-dehydroproline, D,L-3,4-dehydroproline, L-3-hydroxyproline, L-4-hydroxyproline, and L-thiazolidine carboxylic acid derivatives are obtained by substituting the products of Examples 3—12 for the 3-mercapto-2-D-methylpropanoyl-L-proline in Examples 77—85 and substantially following the procedures of Example 77.

An alternative method of preparing $N^{\alpha}$-[3-($N^{\alpha}$-benzoyl-L-phenylalanylthio)-2-D-methylpropanoyl]-L-proline is described in the following two examples.

Example 87

Preparation of $N^{\alpha}$-benzoyl-L-phenylalanine-N-hydroxysuccinimide ester

1.347 g of benzoyl-phenylalanine and 0.576 g of N-hydroxy succinimide were mixed in a 1:1 (by volume) mixture of THF and DMF. The mixture was incubated at 4°C overnight in the presence of 1.133 g of dicyclohexylcarbodiimide.

The reaction mixture was filtered and the solvent was removed under reduced pressure at 30°C. A white residue remained which was recrystallized from THF-isopropyl alcohol to yield 1.194 g (65.2%) of a white solid, m.p. 156°C—157°C. The infrared absorption spectrum in chloroform showed bands at 3440 $cm^{-1}$ indicating an NH group, at 1818 $cm^{-1}$, 1790 $cm^{-1}$, and 1744 $cm^{-1}$, characteristic of the N-carboxy succinimide group and at 1669 $cm^{-1}$, characteristic of the N-benzoyl moiety.

Example 88

Synthesis of $N^{\alpha}$-[3-($N^{\alpha}$-benzoyl-L-phenylalanylthio)-2-D-methylpropanoyl]-L-proline

A reaction mixture containing 93.3 mg of 2-D-methyl-3-mercaptopropanoyl-L-proline, 62 mg of 1-hydroxybenzotriazole (HOBt), 165 mg of N-benzoyl-phenylalanyl-N-hydroxy-succinimide ester, prepared as described in Example 87, was cooled in an ice bath at approximately 0°C, after which 0.0544 ml of N-ethyl morpholine was added. The reaction mixture was stirred in an ice bath for three hours, incubated at 4°C overnight, then at room temperature for twenty hours. The reaction was terminated by the addition of 25 $\mu$l of N,N-dimethyl-1,3-propanediamine, and stirred for an additional two hours. Ethyl acetate was added to the reaction mixture which was then washed by extraction with cold 0.1 N HC1, followed by two washes with water and three washes with saturated NaC1. The mixture was then dried over anhydrous $MgSO_4$ and filtered. The solvent was removed under reduced pressure in a rotary evaporator, yielding a clear, oily product.

The product was purified by chromatography on a column of Sephadex LH-20[tm], 1.2 cm×97 cm. The column was eluted with THF, and 2.5 ml fractions were collected. Fractions 30 and 31 were pooled

and yielded, after solvent removal under high vacuum, 110 mg of product. Anhydrous ether was added and a white gum-like material was obtained. The ether was decanted and the white gum-like material was dissolved in THF and transferred to a vial, dried in a stream of nitrogen, then further dried over $P_2O_5$ overnight. A foam-like product, 82 mg, was obtained.

Fractions 29, 32 and 33 were rechromatographed under identical conditions. Fractions 33 through 36 of the second chromatography were pooled, worked up as described and yielded an additional 51 mg of product. Total yield was 71%.

An alternative method of preparing $N^{\alpha}$-[2-($N^{\alpha}$-benzoyl-L-phenylalanylthio)-propanoyl]-L-3,4-dehydroproline and related derivatives is described in the following two examples.

Example 89

Synthesis of 2-benzoylphenylalanylthiopropanoic acid

A solution of benzoyl-phenylalanine in 30 ml of redistilled dimethylformamide (DMF) is cooled to −20°C. A solution of 1,1′-carbonyldiimidazole in 10 ml redistilled DMF is added dropwise with vigorous stirring. Temperature is not allowed to exceed −14°C. Following the addition, the solution is stirred at −10°C for two hours. D,L-Thiolactic acid in redistilled DMF previously neutralized with redistilled N-ethylmorpholine is then added with continued stirring at −10°C for one hour. The solution is then slowly warmed to room temperature. An approximately equal volume of ethyl acetate is added. The mixture is then cooled and neutralized with concentrated HC1 in saturated NaC1. The organic phase is then washed three times with subsaturated NaC1, i.e., five volumes saturated NaC1 diluted with one volume water. In some cases a three-layer system is observed. In such cases, the middle layer is saved and combined with the lower aqueous phase. The organic phase is dried over anhydrous $MgSO_4$, filtered and placed in the rotary evaporator to remove solvent. The combined aqueous phase and middle phase is acidified at 0°C with concentrated HC1 to pH 2, and extracted three times with ethyl acetate. The organic phase is washed with saturated NaC1 and dried over anhydrous magnesium sulfate, filtered and rotary evaporated. A clear oil is recovered.

Example 90

Synthesis of $N^{\alpha}$-[2-($N^{\alpha}$-benzoyl-L-phenylalanylthiopropanoyl]-L-3,4-dehydroproline

Boc-L-3,4-dehydroproline (213 mg; 1 mmole) was deprotected with 1 ml of anhydrous trifluoroacetic acid for 1 hour. Anhydrous ethyl ether, 3 ml, was added. Solvents were removed using a rotary evaporator at 30°C. The residue was washed three times with ether and then was dried in a vacuum dessicator over NaOH.

A solution of 358 mg (1 mmole) of the product from Example 89 in 3 ml of redistilled DMF was cooled to −20°C in a dry ice-acetone bath. A solution of 1,1′-carbonyldiimidazole, 171 mg (1.05 mmole) in 1 ml of redistilled DMF was added dropwise, and the temperature of the reaction mixture was maintained at −10°C to −15°C. The reaction mixture was stirred for two hours. A cold solution of L-3,4-dehydroproline trifluoroacetate in 2 ml of DMF (neutralized with triethylamine) was added. The reaction mixture was warmed slowly to room temperature. Solvent was removed by rotary evaporation, using high vacuum, at 35°C. The residue was dissolved in 10 ml of ethyl acetate and 2 ml of $H_2O$. The mixture was acidified with 1 N HC1 to pH 2 at 0°C and was mixed. The organic phase was separated and saved. The aqueous phase was extracted three more times with 5 ml of ethyl acetate.

The combined organic phase was washed twice with $H_2O$, three times with saturated NaC1 and then was decolorized with charcoal. The organic phase was dried with anhydrous $MgSO_4$ and then filtered. The filtrate was evaporated to dryness on a rotary evaporator, and an oily product, 366 mg, was obtained. The product was purified by chromatography on Sephadex LH-20$^{tm}$ (1.2×98 cm) was equilibrated and eluted with methanol. Fractions of 150 drops (2.9 ml) were collected. The desired compound was eluted in fractions 24—29. Solvent was removed by rotary evaporation.

Additional purification was achieved by a second chromatography step on Sephadex LH-20$^{tm}$. The second column was equilibrated and eluted with isopropanol, peak fractions were pooled and solvent was removed by rotary evaporation. The product was approximately 95% pure as judged by thin layer chromatography in solvent systems (2) and (3).

Example 91

The inhibitory potency of various of the above-synthesized compounds *in vitro* was measured in the assay system described in Example 1. The enzyme preparation was purified from human urine as described by Ryan, J. W., et al., *Tissue and Cell 10,* 555 (1978). Table 1 shows the $I_{50}$ value for various compounds. The $I_{50}$ value is the concentration of inhibitor required to produce 50% inhibition of the enzyme under a standard assay system containing substrate at a level substantially below $K_m$.

TABLE 1

| Compound | $I_{50}$ |
|---|---|
| Example 13 | $2.6\times10^{-8}$M |
| Example 24 | $3.4\times10^{-8}$M |
| Example 35 | $7.5\times10^{-9}$M |
| Example 45 | $8.8\times10^{-9}$M |
| Example 59 | $2.5\times10^{-8}$M |
| Example 60 | $1.5\times10^{-8}$M |
| Example 77 | $9.4\times10^{-9}$M |
| Example 80 | $2.5\times10^{-8}$M |
| Example 81 | $7.5\times10^{-9}$M |
| Example 90 (racemic) | $3\ \times10^{-9}$M |

Example 92

Oral effectiveness of various compounds

Rats (210—290 g body weight) were fasted overnight and then anesthetized with intraperitoneal pentobarbital, 50—60 mg/kg. Trachesostomy was performed and the animals were ventilated mechanically. A cannula was inserted into a femoral vein for injection of angiotensin I, and a second cannula was inserted into a common carotid artery for direct measurement of arterial blood pressure. Heparin, 1,000 units, was injected via the femoral vein to prevent coagulation. Blood pressure was measured with a pressure transducer connected to a polygraph. The rats were injected with 160—400 ng/kg of angiotensin I in 20 $\mu$l of 0.9 g % NaC1; an amount of angiotensin I sufficient to raise mean arterial blood pressure by 27—50 mm Hg. After the responsiveness of a given rat to angiotensin I was established, the respective compound at 5.5—23 $\mu$mole/kg (drug dissolved in 0.15 ml of $H_2O$ plus 10 $\mu$l of 1 N $NaHCO_3$), was given via a stomach tube. At timed intervals, the effects of 160—400 ng/kg of angiotensin I on mean arterial blood pressure were tested. Results are shown in Table 2. The dose of the particular compound is shown in parenthesis behind the compound number. For example 13 (13.6) indicates that 13.6 $\mu$mole/kg of the compound from Example 13 was administered orally. The time, in minutes, after oral administration of the particular compound when the effects of angiotension I were tested is indicated in brackets behind the "% of control" reading.

TABLE 2

Blood pressure response to 160—400 ng/kg of Angiotensin I expressed as % of control [minutes after oral administration]

Compound (dose)

| 13 (13.6) | 24 (20) | 35 (23) | 45 (20) | 60 (17) | 73 (20) |
|---|---|---|---|---|---|
| 100 [−5] | 100 [−5] | 100 [−5] | 100 [−5] | 100 [−5] | 100 [−5] |
| 40 [+3] | 100 [+3] | 71 [+2] | 77 [+6] | 59 [+5] | 53 [+1] |
| 40 [7] | 43 [11] | 63 [6] | 69 [10] | 44 [10] | 66 [5] |
| 28 [13] | 27 [22] | 49 [16] | 46 [15] | 44 [15] | 26 [15] |
| 24 [18] | 24 [29] | 43 [23] | 38 [25] | 44 [31] | 26 [18] |
| 20 [23] | 22 [34] | 43 [37] | 38 [29] | 38 [37] | 21 [21] |
| 18 [31] | 19 [51] | 40 [48] | 33 [38] | 44 [45] | 21 [25] |
| 20 [34] | | 26 [64] | 33 [50] | 44 [55] | 24 [29] |
| 20 [42] | | 26 [71] | 31 [57] | 36 [81] | 18 [39] |
| 16 [48] | | 37 [84] | 31 [65] | 36 [93] | 16 [44] |
| 16 [58] | | 37 [96] | 26 [74] | 38 [104] | 16 [49] |
| 20 [67] | | 40 [109] | 36 [88] | | 18 [54] |
| 20 [80] | | 54 [124] | | | 18 [60] |
| 18 [90] | | 51 [140] | | | 18 [74] |
| 16 [100] | | 63 [157] | | | 21 [86] |
| 20 [112] | | 77 [171] | | | 21 [110] |
| 20 [125] | | | | | 26 [120] |
| 30 [137] | | | | | 26 [132] |
| 50 [167] | | | | | 26 [142] |

TABLE 2 (contd.)
Blood pressure response to 160—400 ng/kg of angiotensin I expressed as % of control [minutes after oral administration]

Compound (dose)

| 77 (20) | 80 (11) | 80 (5.5) | 81 (7.6) | 90 (23) |
|---|---|---|---|---|
| 100 [−5] | 100 [−6] | 100 [−6] | 100 [−5] | 100 [−5] |
| 92 [+3] | 67 [+6] | 111 [+6] | 95 [+1] | 100 [+3] |
| 64 [7] | 57 [12] | 93 [12] | 95 [6] | 81 [9] |
| 32 [21] | 43 [24] | 81 [18] | 95 [11] | 74 [14] |
| 40 [26] | 37 [35] | 85 [24] | 92 [22] | 74 [24] |
| 40 [32] | 17 [48] | 56 [35] | 68 [28] | 74 [28] |
| 20 [38] | 17 [58] | 56 [58] | 61 [33] | 63 [34] |
| 8 [50] | 20 [68] | 52 [68] | 58 [39] | 56 [44] |
| 8 [56] | 17 [78] | 30 [88] | 47 [45] | 44 [54] |
| 8 [70] | 13 [88] | 37 [118] | 47 [60] | 56 [64] |
| 20 [79] | 27 [185] | 60 [138] | 45 [67] | 37 [85] |
| 12 [87] | 33 [205] | 37 [169] | 47 [74] | 44 [94] |
| 12 [104] | 33 [213] | 37 [185] | 47 [82] | 74 [104] |
| 20 [114] | 43 [226] | 44 [205] | 47 [92] | 56 [114] |
| 40 [124] | | | 55 [137] | 100 [146] |
| 40 [136] | | | 55 [142] | |
| 36 [148] | | | 47 [152] | |
| 48 [173] | | | 63 [185] | |
| 48 [186] | | | 79 [207] | |
| 68 [199] | | | 74 [230] | |
| 64 [212] | | | | |
| 64 [226] | | | | |
| 80 [242] | | | | |
| 80 [260] | | | | |
| 84 [291] | | | | |

Example 93

Intraveneous effectiveness of various compounds

Anesthetized rats were prepared as described in Example 92. After the responsiveness of a given rat to angiotensin I was established, the respective compound, at 2 μmole/kg in a volume of 15 μl of 0.01N sodium bicarbonate, was injected via a femoral vein. At timed intervals, the effects of angiotensin I, 400 ng/kg, on mean arterial blood pressure were tested. Results are shown in Table 3. Table 3 follows the same format as Table 2.

TABLE 3
Blood pressure response to 400 ng/kg of angiotensin I as % of control
[minutes after intraveneous administration]

| Compound (dose) | | |
|---|---|---|
| 24 (2) | 60 (2) | 77 (2) |
| 100%[−5] | 100%[−5] | 100%[−5] |
| 0 [+0.5] | 8 [+3] | 8 [+0.5] |
| 19 [3] | 15 [9] | 18 [5] |
| 19 [9] | 23 [14] | 21 [11] |
| 22 [13] | 23 [19] | 24 [14] |
| 31 [18] | 33 [24] | 29 [17] |
| 31 [22] | 41 [37] | 34 [20] |
| 50 [34] | 46 [45] | 42 [24] |
| 63 [47] | 46 [54] | 47 [32] |
| 78 [65] | 46 [77] | 53 [43] |
| 75 [103] | 51 [87] | 58 [49] |
| 94 [113] | | 61 [56] |
| | | 71 [71] |
| | | 78 [76] |
| | | 76 [88] |
| | | 76 [99] |
| | | 74 [109] |
| | | 84 [125] |

While the invention has been described in connection with specific embodiments thereof, it will be understood that it is capable of further modifications and this application is intended to cover any variations, uses, or adaptations of the invention following, in general, the principles of the invention and including such departures from the present disclosure as come within known or customary practice within the art to which the invention pertains and as may be applied to the essential features hereinbefore set forth, and as follows in the scope of the appended claims.

**Claims**

1. An angiotensin converting enzyme inhibitor having the formula:

$$R{-}A{-}S{-}(CH_2)_n{-}\underset{\displaystyle R_1}{\underset{|}{CH}}{-}\overset{\displaystyle O}{\overset{\|}{C}}{-}R_2,$$

wherein

R is hydrogen, formyl, acetyl, propanoyl, butanoyl, phenylacetyl, phenylpropanoyl, benzoyl, cyclopentylcarbonyl, tert-butyloxycarbonyl, cyclopentylcarbonyl-L-lysyl, pyro-L-glutamyl-L-lysyl, L-arginyl, L-lysyl or pyro-L-glutamyl;

A is L-phenylalanyl, glycyl, L-alanyl, L-tryptophyl, L-tyrosyl, L-isoleucyl, L-leucyl, L-histidyl or L-valyl, the $\alpha$-amino group thereof being in amide linkage with R;

$R_1$ is hydrogen or methyl;

$R_2$ is L-proline, L-3,4-dehydroproline, D,L-3,4-dehydroproline, L-3-hydroxyproline, L-4-hydroxyproline or L-thiazolidine carboxylic acid, the imino group thereof being in imide linkage with the adjacent

$$-\overset{\displaystyle O}{\overset{\|}{C}}-;$$

and,

n is 0 or 1, such that when n=0, $R_1$ is methyl.

2. The inhibitor of claim 1 wherein R is benzoyl.

3. The inhibitor of claim 1 wherein R is hydrogen.

4. The inhibitor of claim 1 wherein R is formyl acetyl, propanoyl, butanoyl, phenylacetyl or phenylpropanoyl.

5. The inhibitor of claim 1 wherein R is cyclopentylcarbonyl or tert-butyloxycarbonyl.

6. The inhibitor of claim 1 wherein R is cyclopentylcarbonyl-L-lysyl or pyro-L-glutamyl-L-lysyl.

7. The inhibitor of claim 1 wherein R is L-arginyl, L-lysyl or pyro-L-glutamyl.

8. The inhibitor of claim 1 wherein R is benzoyl, A is L-phenylalanyl, $R_1$ is methyl, $R_2$ is L-proline and n=1.

9. An inhibitor as defined in any of claims 1—8 for use in inhibiting angiotensin *in vivo*.

10. An inhibitor as defined in any of claims 1 to 8 for use in reducing blood pressure.

11. A process for preparing an angiotensin converting enzyme inhibitor useful for treating hypertension having the formula

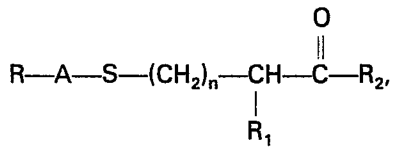

wherein

R is formyl, acetyl, propanoyl, butanoyl, phenylacetyl, phenylpropanoyl, benzoyl, cyclopentylcarbonyl, tert-butyloxycarbonyl, cyclopentylcarbonyl-L-lysyl, pyro-L-glutamyl-L-lysyl, L-arginyl, L-lysyl or pyro-L-glutamyl;

A is L-phenylalanyl, glycyl, L-alanyl, L-tryptophyl, L-tyrosyl, L-isoleucyl, L-leucyl, L-histidyl or L-valyl, the $\alpha$-amino group thereof being in amide linkage with R;

$R_1$ is hydrogen or methyl;

$R_2$ is L-proline, L-3,4-dehydroproline, D,L-3,4-dehydroproline, L-3-hydroxyproline, L-4-hydroxyproline or L-thiazolidine carboxylic acid, the imino group thereof being in imide linkage with the adjacent

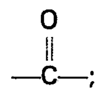

and

n is 0 or 1, such that when n=0, $R_1$ is methyl, which comprises the following steps:

(1) reacting

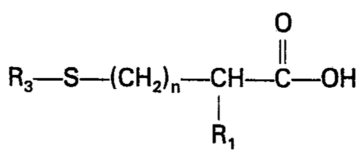

with $R_2$ to give

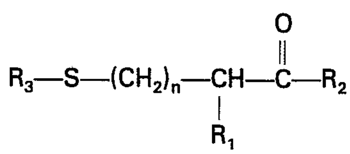

wherein

$R_3$ is acetyl and $R_1$, $R_2$ and n are defined as above;

(2) removing $R_3$ from the product of step (1) to give

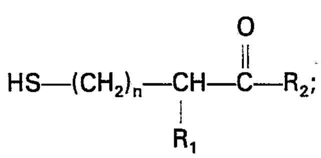

(3) reacting the product of step (2) with R—A to give

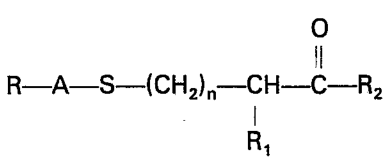

wherein

R, A, $R_1$, $R_2$ and n are defined as above.

12. The process of claim 11 wherein the reaction described in step (1) is conducted either in the presence of 1,1′-carbonyldiimidazole or dicyclohexylcarbodiimide and the reaction described in step (3) is conducted in the presence of 1,1′-carbonyldiimidazole.

13. The process of claim 12 wherein the $R_2$ in step (1) also has a protecting group for the carboxyl which is removed in step (2).

14. The process of claim 12 wherein R is benzoyl.

15. The process of claim 12 wherein R is formyl, acetyl, propanoyl, butanoyl, phenylacetyl and phenylpropanoyl.

16. The process of claim 12 wherein R is cyclopentylcarbonyl or tert-butyloxycarbonyl.

17. The process of claim 12 wherein R is cyclopentylcarbonyl-L-lysyl or pyro-L-glutamyl-L-lysyl.

18. The progress of claim 12 wherein R is L-arginyl, L-lysyl or pyro-L-glutamyl.

19. The process of claim 16 wherein

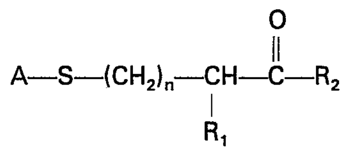

is formed by deprotecting tert-butyloxycarbonyl-

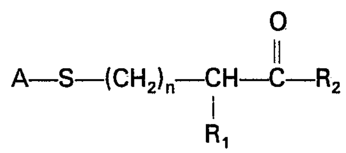

wherein

A, $R_1$, $R_2$ and n are as defined above.

20. The process of claim 12 wherein R is benzoyl, A is L-phenylalanyl, $R_1$ is methyl, $R_2$ is L-proline and n=1.

**Patentansprüche**

1. Ein Inhibitor des Angiotension umwandelnden Enzyms mit der Formel:

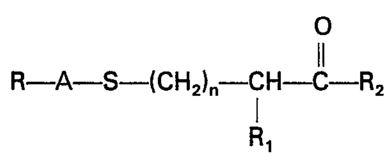

worin R Wasserstoff, Formyl, Acetyl, Propanoyl, Butanoyl, Phenylacetyl, Phenylpropanoyl, Benzoyl, Cyclopentylcarbonyl, tert.-Butyloxycarbonyl, Cyclopentylcarbonyl-L-lysyl, Pyro-L-glutamyl-L-lysyl, L-Arginyl, L-Lysyl oder Pyro-L-glutamyl bedeutet; A L-Phenylalanyl, Glycyl, L-Alanyl, L-Tryptophyl, L-Tyrosyl, L-Isoleucyl, L-Leucyl, L-Histidyl oder L-Valyl bedeutet, wobei deren $\alpha$-Aminogruppe in Amidbindung mit R verbunden ist;

$R_1$ Wasserstoff oder Methyl darstellt;

$R_2$ L-Prolin, L-3,4-Dehydroprolin, D,L-3,4-Dehydroprolin, L-3-Hydroxyprolin, L-4-Hydroxyprolin oder L-Thiazolidincarbonsäure bedeutet, wobei deren Iminogruppe in Imidbindung mit der benachbarten Gruppe

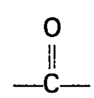

verbunden ist und

n o oder 1 bedeutet, wobei $R_1$ Methyl ist, wenn n=0 ist.

2. Der Inhibitor gemäß Anspruch 1, worin R Benzoyl bedeutet.

3. Der Inhibitor gemäß Anspruch 1, worin R Wasserstoff bedeutet.

4. Der Inhibitor gemäß Anspruch 1, worin R Formyl, Acetyl, Propanoyl, Butanoyl, Phenylacetyl oder Phenylpropanoyl bedeutet.

5. Der Inhibitor gemäß Anspruch 1, worin R Cyclopentylcarbonyl oder tert.-Butyloxycarbonyl bedeutet.

6. Der Inhibitor gemäß Anspruch 1, worin R Cyclopentylcarbonyl-L-lysyl oder Pyro-L-glutamyl-L-lysyl bedeutet.

7. Der Inhibitor gemäß Anspruch 1, worin R L-Arginyl, L-Lysyl oder Pyro-L-glutamyl bedeutet.

8. Der Inhibitor gemäß Anspruch 1, worin R Benzoyl bedeutet, A L-Phenylalanyl ist, $R_1$ Methyl bedeutet, $R_2$ L-Prolin ist und n=1 ist.

9. Ein Inhibitor gemäß einem der Ansprüche 1 bis 8 zur Verwendung bei der Hemmung von Angiotensin in vivo.

10. Ein Inhibitor gemäß einem der Ansprüche 1 bis 8 zur Verwendung bei der Verringerung des Blutdruckes.

11. Ein Verfahren zur Herstellung eines Inhibitors des Angiotensin umwandelnden Enzyms, der zur Behandlung von hohem Blutdruck brauchbar ist, mit der Formel

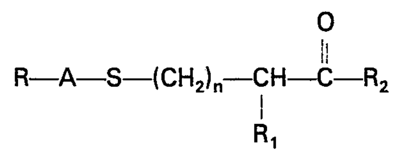

worin R Formyl, Acetyl, Propanoyl, Butanoyl, Phenylacetyl, Phenylpropanyl, Benzoyl, Cyclopentylcarbonyl, tert.-Butyloxycarbonyl, Cyclopentylcarbonyl-L-lysyl, Pyro-L-glutamyl-L-lysyl, L-Arginyl, L-Lysyl oder Pyro-L-glutamyl bedeutet;

A L-Phenylalanyl, Glycyl, L-Alanyl, L-Tryptophyl, L-Tyrosyl, L-Isoleucyl, L-Leucyl, L-Histidyl oder L-Valyl bedeutet, deren $\alpha$-Aminogruppe in Amidbindung mit R verbunden ist;

$R_1$ Wasserstoff oder Methyl darstellt;

$R_2$ L-Prolin, L-3,4-Dehydroprolin, D,L-3,4-Dehydroprolin, L-3-Hydroxyprolin, L-4-Hydroxyprolin oder L-Thiazolidincarbonsäure bedeutet, deren Iminogruppe in Imidbindung mit der benachbarten Gruppe

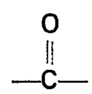

verbunden ist; und

n 0 oder 1 bedeutet, wobei $R_1$ Methyl bedeutet, wenn n=0 ist, welches die folgenden Stufen umfaßt:

(1) Umsetzung von

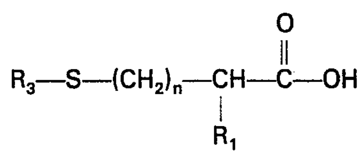

mit $R_2$ unter Bildung von

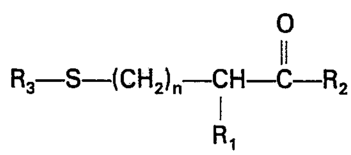

worin $R_3$ Acetyl bedeutet und $R_1$, $R_2$ und n die vorstehende Bedeutung aufweisen;

(2) Entfernung von $R_3$ aus dem Produkt aus Stufe (1) unter Bildung von

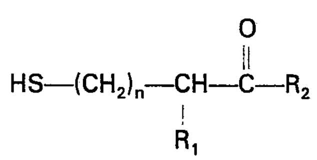

(3) Umsetzung des Produktes aus Stufe (2) mit R—A unter Bildung von

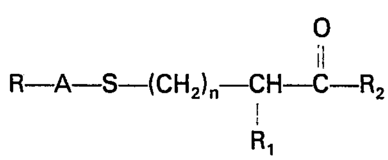

worin R, A, $R_1$, $R_2$ und n die vorstehenden Bedeutungen aufweisen.

12. Das Verfahren gemäß Anspruch 11, worin die in Stufe (1) beschriebene Umsetzung entweder in Gegenwart von 1,1'-Carbonyldiimidazol oder von Dicyclohexylcarbodiimid durchgeführt wird und die in Stufe (3) beschriebene Umsetzung in Gegenwart von 1,1'-Carbonyldiimidazol durchgeführt wird.

13. Das Verfahren gemäß Anspruch 12, worin das $R_2$ in Stufe (1) auch eine Schutzgruppe für die Carboxylgruppe aufweist, die in Stufe (2) entfernt wird.

14. Das Verfahren gemäß Anspruch 12, worin R Benzoyl ist.

15. Das Verfahren gemäß Anspruch 12, worin R Formyl, Acetyl, Propanoyl, Butanoyl, Phenylacetyl und Phenylpropanoyl bedeutet.

16. Das Verfahren gemäß Anspruch 12, worin R Cyclopentylcarbonyl oder tert.-Butyloxycarbonyl bedeutet.

17. Das Verfahren gemäß Anspruch 12, worin R Cyclopenylcarbonyl-L-lysyl oder Pyro-L-glutamyl-L-lysyl bedeutet.

18. Das Verfahren gemäß Anspruch 12, worin R L-Arginyl, L-Lysyl oder Pyro-L-glutamyl bedeutet.

19. Das Verfahren gemäß Anspruch 16, worin

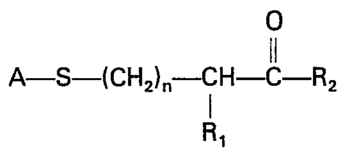

durch Abspalten der Schutzgruppe aus tert.-Butyloxycarbonyl-

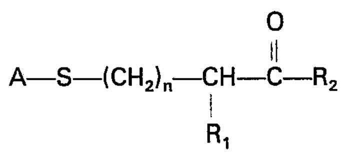

worin A, $R_1$, $R_2$ und n die vorstehenden Bedeutungen aufweisen, gebildet wird.

20. Das Verfahren gemäß Anspruch 12, worin R Benzoyl bedeutet, A L-Phenylalanyl darstellt, $R_1$ Methyl bedeutet, $R_2$ L-Prolin bedeutet und n=1 ist.

## Revendications

1. Inhibiteur de l'enzyme de conversion de l'angiotensine répondant à la formule:

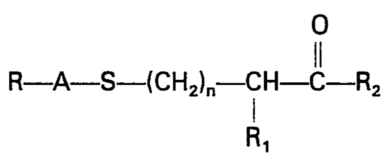

dans laquelle:

R est l'hydrogène, un groupe formyle, acétyle, propanoyle, butanoyle, phénylacétyle, phénylpropanoyle, benzoyle, cyclopentylcarbonyle, tert-butyloxycarbonyle, cyclopentylcarbonyl-L-lysyle, pyro-L-glutamyl-L-lysyle, L-arginyle, L-Lysyle ou pyro-L-glutamyle;

A est un groupe L-Phénylalanyle, glycyle, L-alanyle, L-tryptophyle, L-tyrosyle, L-isoleucyle, L-leucyle, L-histidyle ou L-valyle, dont le groupe $\alpha$-amino forme une liaison amide avec R;

$R_1$ est l'hydrogène ou un groupe méthyle;

$R_2$ est la L-proline, la L-3,4-déshydroproline, la D,L-3,4-déshydroproline, la L-3-hydroxyproline, la L-4-hydroxyproline ou l'acide L-thiazolidine carboxylique, dont le groupe imino forme une liaison imide avec le groupe adjacent

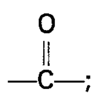

et

n est 0 ou 1, de telle sorte que lorsque n=0, $R_1$ est un groupe méthyle.

2. Inhibiteur selon la revendication 1, dans lequel R est un groupe benzoyle.

3. Inhibiteur selon la revendication 1, dans lequel R est l'hydrogène.

4. Inhibiteur selon la revendication 1, dans lequel R est un groupe formyle, acétyle, propanoyle, butanoyle, phénylacétyle ou phénylpropanoyle.

5. Inhibiteur selon la revendication 1, dans lequel R est un groupe cyclopentylcarbonyl ou tert-butyloxycarbonyle.

6. Inhibiteur selon la revendication 1, dans lequel R est un groupe cyclopentylcarbonyl-L-lysyle ou pyro-L-glutamyl-L-lysyle.

7. Inhibiteur selon la revendication 1, dans lequel R est un groupe L-arginyle, L-lysyle ou pyro-L-glutamyle.

8. Inhibiteur selon la revendication 1, dans lequel R est un groupe benzoyle, A est un groupe L-phénylalanyle, $R_1$ est un groupe méthyle, $R_2$ est la L-proline et n=1.

9. Inhibiteur selon l'une quelconque des revendications 1 à 8, utilisable dans l'inhibition in vivo de l'angiotensine.

10. Inhibiteur selon l'une quelconque des revendications 1 à 8, utilisable pour réduire la tension sanguine.

11. Procédé pour la préparation d'un inhibiteur de l'enzyme de conversion de l'angiotensine, utilisable pour le traitement de l'hypertension, répondant à la formule:

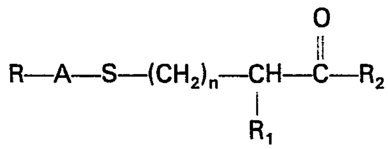

dans laquelle:

R est un groupe formyle, acétyle, propanoyle, butanoyle, phénylacétyle, phénylpropanoyle, benzoyle, cyclopentylcarbonyle, tert-butyloxycarbonyle, cyclopentylcarbonyllysyle, pyro-L-glutamyl-L-lysyle, L-arginyle, L-lysyle ou pyro-L-glutamyle;

A est un groupe L-phénylalanyle, glycyle, L-alanyle, L-tryptophyle, L-tyrosyle, L-isoleucyle, L-leucyle, H-histidyle ou L-valyle, dont le groupe $\alpha$-amino forme une liaison amide avec R;

$R_1$ est l'hydrogène ou un groupe méthyle;

$R_2$ est la L-proline, la L-3,4-déshydroproline, la D,L-3,4-déshydroproline, la L-3-hydroxyproline, la L-4-hydroxyproline ou l'acide L-thiazolidine carboxylique, dont le groupe imino forme une liaison imide avec le groupe adjacent

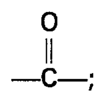

et n est 0 ou 1, de telle sorte que lorsque n=0, $R_1$ est une groupe méthyle,

comportant les opérations suivantes:

(1) réaction de

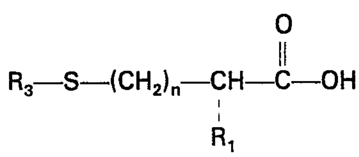

avec $R_2$ pour obtenir

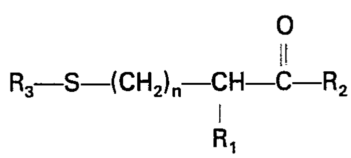

dans lequel $R_3$ est un groupe acétyle et $R_1$, $R_2$ et n sont tels que définis ci-dessus;

(2) élimination de $R_3$ du produit du stade (1) pour obtenir

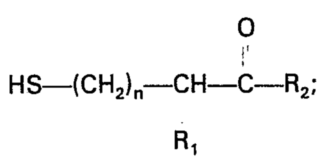

(3) réaction du produit du stade (2) avec R—A pour obtenir

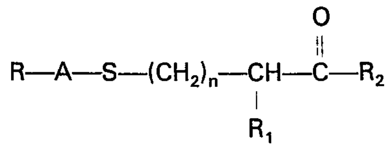

où R, A, $R_1$, $R_2$ et n sont tels qui définis ci-dessus.

12. Procédé selon la revendication 11, caractérisé en ce que la réaction décrite au stade (1) est effectuée en présence de 1,1'-carbonyldiimidazole ou de dicyclohexylcarbodiimide et la réaction décrite au stade (3) est effectuée en présence de 1,1'-carbonyldiimidazole.

13. Procédé selon la revendication 12, caractérisé en ce que $R_2$ au stade (1) comporte également un groupe protecteur pour le groupe carboxyle, qui est éliminé au stade (2).

14. Procédé selon la revendication 12, dans lequel R est un groupe benzoyle.

15. Procédé selon la revendication 12, dans lequel R est un groupe formyle, acétyle, propanoyle, butanoyle, phénylacétyle et phénylpropanoyle.

16. Procédé selon la revendication 12, dans lequel R est un groupe cyclopentylcarbonyle ou tert-butyloxycarbonyle.

17. Procédé selon la revendication 12, dans lequel R est un groupe cyclopentylcarbonyl-L-lysyle ou pyro-L-glutamyl-L-lysyle.

18. Procédé selon la revendication 12, dans lequel R est un groupe L-arginyle, L-lysyle ou pyro-L-glytamyle.

19. Procédé selon la revendication 16, caractérisé en ce que

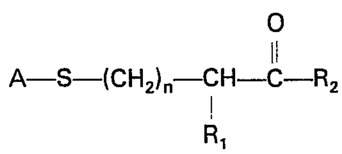

est formé en éliminant le groupe protecteur à partir du composé tert-butyloxycarbonyl-

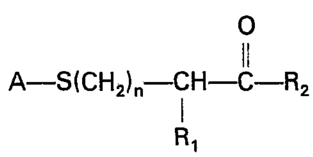

dans lequel A, $R_1$, $R_2$ et n sont tels que définis ci-dessus.

20. Procédé selon la revendication 12, dans lequel R est un groupe benzoyle, A est un groupe L-phénylalanyle, $R_1$ est un groupe méthyle, $R_2$ est la L-proline et n=1.